# FASCICULE DE BREVET EUROPEEN

(11) **EP 3 389 779 B1**
(45) Date de publication et mention de la délivrance du brevet: **18.09.2019**
(21) Numéro de dépôt: 16825460.5
(22) Date de dépôt: 07.12.2016
(51) Int. Cl.: A61N 5/06

(54) **DISPOSITIF IMPLANTABLE POUR LA STIMULATION OPTIQUE DU CERVEAU**
IMPLANTIERBARE VORRICHTUNG ZUR OPTISCHEN STIMULATION DES GEHIRNS
IMPLANTABLE DEVICE FOR OPTICAL STIMULATION OF THE BRAIN

(30) Priorité: 17.12.2015 FR 1562698
(43) Date de publication de la demande: 24.10.2018
(73) Titulaire: Commissariat à l'Energie Atomique et aux Energies Alternatives, 75015 Paris (FR)
(72) Inventeur: CHABROL, Claude, 38320 Poisat (FR); BENABID, Alim-Louis, 38240 Meylan (FR)
(74) Mandataire: Cabinet Beaumont
(86) Numéro de dépôt international: PCT/FR2016/053248
(87) Numéro de publication internationale: WO 2017/103380

(56) Documents cités:
- WO-A1-2009/155371
- FR-A1- 3 010 321
- US-A- 5 782 896
- US-A1- 2009 118 800

## Description

### Domaine

La présente demande concerne le domaine des dispositifs implantables pour la stimulation, par irradiation optique, d'organes du corps humain ou animal, et en particulier pour la stimulation optique profonde du cerveau.

### Exposé de l'art antérieur

La stimulation cérébrale profonde ("deep brain stimulation" en anglais) est une technique thérapeutique comportant l'implantation dans le cerveau d'un patient d'un dispositif permettant de stimuler des parties spécifiques du cerveau. On a notamment proposé des traitements de certains dysfonctionnements neuronaux, dont la maladie de parkinson, par irradiation optique de parties du cerveau avec une source de lumière émettant dans le domaine de l'infrarouge.

On a déjà proposé des dispositifs implantables permettant de mettre en oeuvre des traitements par irradiation optique profonde du cerveau au moyen d'une fibre optique introduite dans le cerveau du patient, par l'intermédiaire de laquelle de la lumière provenant d'une source de lumière placée à l'extérieur du cerveau est guidée vers le cerveau. Un exemple d'un tel dispositif est décrit dans la demande de brevet FR3010321 précédemment déposée par le demandeur.

L'utilisation de fibres optiques présente toutefois des limitations. En particulier, une fibre optique ne permet d'éclairer qu'une surface relativement restreinte du cerveau, en raison de la forte directivité de la lumière qu'elle projette à sa sortie. En outre, les pertes de couplage dans la fibre optique obligent à utiliser une source lumineuse adaptée (par exemple laser, de préférence à LED), ce qui se traduit par une consommation électrique relativement élevée du dispositif.

Un autre exemple de dispositif implantable permettant de mettre en oeuvre des traitements par irradiation optique est décrit dans le document US5782896.

Il serait souhaitable de pouvoir disposer d'un dispositif pour l'irradiation optique profonde du cerveau, ce dispositif palliant tout ou partie des inconvénients des dispositifs connus.

### Résumé

L'invention est définie dans les revendications attenantes. Ainsi, un mode de réalisation prévoit un dispositif implantable pour la stimulation optique d'un organe du corps humain ou animal, comportant une sonde comprenant un tube en un premier matériau transparent, ce tube étant hermétiquement fermé par au moins un bouchon ; au moins une source lumineuse disposée à l'intérieur du tube ; une pluralité d'éléments de connexion électrique traversant le bouchon et reliant électriquement ladite au moins une source lumineuse à l'extérieur du tube ; et un câble de liaison disposé à l'extérieur du tube, connecté audits éléments de connexion.

Selon un mode de réalisation, le dispositif comporte en outre une gaine en un deuxième matériau transparent enrobant le tube et le câble.

Selon un mode de réalisation, le deuxième matériau est un matériau élastomère.

Selon un mode de réalisation, le premier matériau est du saphir ou de la silice.

Selon un mode de réalisation, la sonde a une forme longiligne, le tube étant situé à une première extrémité de la sonde et le câble de liaison reliant la source lumineuse à une deuxième extrémité de la sonde opposée à la première extrémité.

Selon un mode de réalisation, le dispositif comporte en outre un module d'alimentation et de contrôle relié électriquement à la source lumineuse par l'intermédiaire du câble de liaison.

Selon un mode de réalisation, la source lumineuse comprend une ou plusieurs diodes électroluminescentes.

Selon un mode de réalisation, le dispositif comporte en outre un capteur adapté à détecter une éventuelle fuite dans le tube.

Selon un mode de réalisation, le capteur comprend une diode électroluminescente organique située à l'intérieur du tube.

Selon un mode de réalisation, le capteur comprend en outre un circuit de mesure d'une grandeur représentative de l'impédance de la diode électroluminescente organique et/ou du rendement lumineux de la diode électroluminescente organique.

Selon un mode de réalisation, un matériau de remplissage transparent est disposé à l'intérieur du tube.

Selon un mode de réalisation, la sonde comprend en outre des électrodes adaptées à la mise en oeuvre d'une stimulation électrique d'un organe du corps humain ou animal.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 est une représentation schématique d'un exemple d'un mode de réalisation d'un dispositif implantable pour l'irradiation optique du cerveau ;
la figure 2 illustre plus en détail une partie du dispositif de la figure 1 ; et
la figure 3 représente une variante de réalisation du dispositif décrit en relation avec les figures 1 et 2.

### Description détaillée

De mêmes éléments ont été désignés par de mêmes références aux différentes figures et, de plus, les diverses figures ne sont pas tracées à l'échelle. Par souci de clarté, seuls les éléments qui sont utiles à la compréhension des modes de réalisation décrits ont été représentés et sont détaillés. Sauf précision contraire, les expressions "approximativement", "sensiblement", et "de l'ordre de" signifient à 10 % près, de préférence à 5 % près.

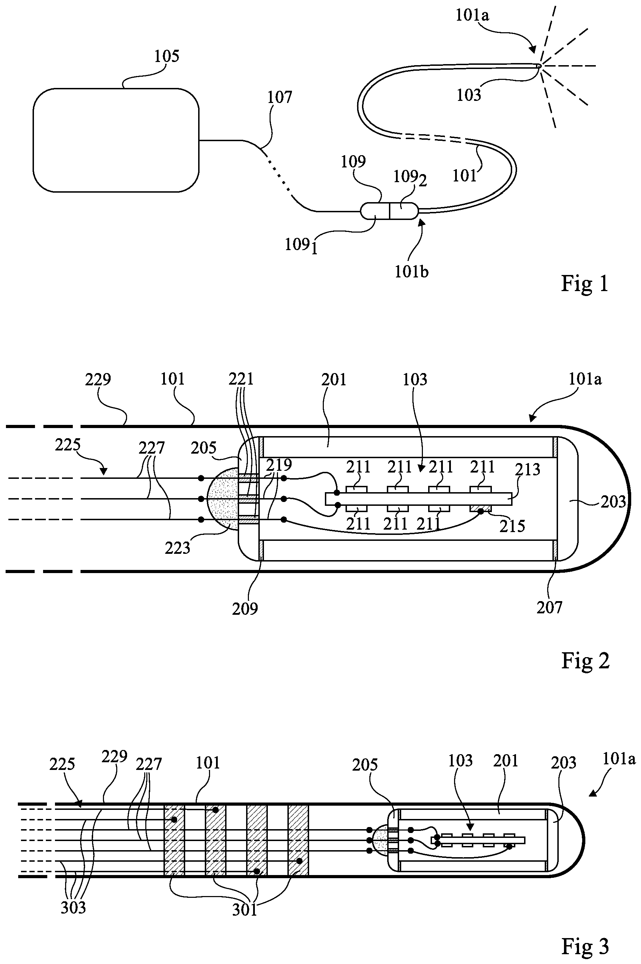

La figure 1 est une représentation schématique d'un exemple d'un mode de réalisation d'un dispositif implantable pour l'irradiation optique du cerveau. Ce dispositif comporte une sonde 101 de forme longiligne dont une première extrémité ou extrémité distale 101a est destinée à être implantée à l'intérieur du cerveau en vis-à-vis d'une partie du cerveau que l'on souhaite stimuler. La sonde 101 a par exemple la forme générale d'une tige, par exemple une tige à section circulaire. La sonde 101 a par exemple la forme générale et les dimensions d'une sonde de type DBS (de l'anglais "Deep Brain Stimulation"), outil couramment utilisé dans le domaine des implantations intracérébrales. A titre d'exemple, la largeur de la sonde 101 (c'est-à-dire son diamètre dans le cas d'une tige à section circulaire) est comprise entre 0,5 et 5 mm et de préférence entre 1,3 et 2,5 mm, et sa longueur est comprise entre 5 cm et 1 m et de préférence entre 15 et 30 cm. La sonde 101 est de préférence souple ou flexible pour limiter les risques de lésion/détérioration des tissus. En pratique, un outil rigide peut être utilisé pour la mise en place chirurgicale de la sonde. La sonde 101 comprend, du côté de son extrémité distale 101a, une source lumineuse 103 adaptée à irradier des portions du cerveau situées en vis-à-vis de l'extrémité distale de la sonde. La sonde 101 comprend en outre un câble de liaison (câble 225 des figures 2 et 3, non visible sur la figure 1) reliant électriquement la source lumineuse 103 à une deuxième extrémité ou extrémité proximale 101b de la sonde.

Le dispositif de la figure 1 comprend de plus un module 105 d'alimentation et de commande de la source lumineuse 103, comportant par exemple une batterie électrique et un circuit de contrôle adapté à commander la source lumineuse 103 pour mettre en oeuvre des stimulations souhaitées du cerveau. Dans l'exemple représenté, le dispositif comporte en outre un câble 107 reliant le module 105 à l'extrémité proximale 101b de la sonde 101. Plus particulièrement, l'extrémité du câble 107 opposée au module 105 est couplée ou connectée électriquement à l'extrémité proximale de la sonde 101, de manière à permettre au module 105 d'alimenter et de commander la source 103.

En pratique, pour minimiser les risques pour le patient, seule la sonde 101 est effectivement implantée dans le cerveau du patient, les autres éléments du dispositif étant maintenus à l'extérieur du cerveau pendant toute la durée du traitement. A titre d'exemple, les éléments du dispositif de la figure 1 autres que la sonde 101 peuvent être implantés dans d'autres parties du corps du patient. Le module d'alimentation et de contrôle 105 peut par exemple être implanté au niveau du thorax du patient. Le couplage entre le câble 107 et la sonde 101 est de préférence détachable ou amovible de façon à pouvoir effectuer certaines opérations de maintenance du module 105 ou du câble 107 sans avoir à extraire la sonde 101 du cerveau du patient. Dans l'exemple représenté, le couplage entre le câble 107 et la sonde 101 est effectué par l'intermédiaire d'un connecteur 109, ce connecteur comportant une partie 109₁ montée solidaire du câble 107 et une partie 109₂ montée solidaire de la sonde 101. Les parties 109₁ et 109₂ du connecteur 109 sont adaptées à coopérer de manière détachable ou amovible pour établir une connexion électrique entre le câble 107 et le câble de liaison de la sonde 101. Le connecteur 109 peut par exemple être implanté sous le cuir chevelu ou dans un logement préalablement fraisé dans la boîte crânienne du patient.

La figure 2 représente plus en détail un exemple de réalisation de la sonde 101 du dispositif de la figure 1. La figure 2 représente plus particulièrement la partie distale de la sonde 101.

La sonde 101 comporte, du côté de son extrémité distale 101a, un tube 201 en un matériau biocompatible transparent, par exemple du saphir ou de la silice, à l'intérieur duquel est disposée la source lumineuse 103. Le tube 201 présente par exemple une section circulaire. A titre d'exemple, la largeur du tube 201 (c'est-à-dire son diamètre dans le cas d'un tube à section circulaire) est comprise entre 1 et 2 mm, et sa longueur est comprise entre 1 et 10 mm. Le tube 201 est par exemple disposé de façon que son axe central longitudinal coïncide sensiblement avec l'axe central longitudinal de la sonde. Le tube 201 peut être réalisé en un matériau rigide, par exemple un matériau présentant un module de Young supérieur à 10 GPa. Le tube transparent 201 est hermétiquement fermé à ses extrémités par des bouchons 203 et 205. Les bouchons 203 et 205 sont par exemple (mais non nécessairement) transparents. Le bouchon 203 peut être intégré au tube 201 de manière à former une seule pièce avec le tube 201. A titre d'exemple, les bouchons 203 et 205 sont en le même matériau biocompatible transparent que le tube 201 de façon à faciliter l'assemblage, par exemple par soudure laser ou brasure métallique. Dans l'exemple représenté, le bouchon 203 est tourné vers la partie distale 101a de la sonde, et le bouchon 205 est tourné vers la partie proximale 101b de la sonde. Le bouchon 203 est hermétiquement fixé sur toute sa périphérie à une première extrémité du tube 201, par exemple par soudure, ou par une brasure hermétique biocompatible 207, par exemple une brasure à l'or. Le bouchon 205 est hermétiquement fixé sur toute sa périphérie à l'extrémité opposée du tube 201, par exemple par soudure ou par une brasure hermétique biocompatible 209, par exemple une brasure à l'or. La fixation des bouchons 203 et 205 aux extrémités du tube 201 est par exemple réalisée par soudure thermosonique ou par thermocompression. Ces méthodes présentent l'avantage de pouvoir être réalisées à relativement basse température, par exemple à une température inférieure à 200°C, ce qui permet de ne pas endommager les composants placés à l'intérieur du tube 201. A titre de variante, la fixation des bouchons 203 et 205 aux extrémités du tube 201 se fait par soudure laser à travers le matériau et sans apport de matière. Une fois les bouchons 203 et 205 en place, l'ensemble comprenant le tube 201 et les bouchons 203 et 205 forme un boîtier hermétique adapté à isoler l'ensemble des composants non biocompatibles qu'il contient, par exemple un boîtier présentant un taux de fuite à l'hélium inférieur à 10⁻⁶ atm.cm³/s et de préférence inférieur à 10⁻⁸ atm.cm³/s, avec 1 atm = 101325 Pa. A titre d'exemple, le boîtier est fermé sous atmosphère neutre (sans oxygène), par exemple sous argon.

Préalablement à la fermeture hermétique du tube 201, la source lumineuse 103 de la sonde est disposée à l'intérieur du tube 201. La source lumineuse comprend un ou plusieurs émetteurs de lumière 211. Les émetteurs 211 peuvent être des diodes laser, par exemple des diodes laser à cavité verticale à émission par la surface (VCSEL), des diodes électroluminescentes (LED), par exemple des diodes électroluminescentes organiques (OLED), ou toutes autres sources lumineuses adaptées. Dans l'exemple représenté, les émetteurs 211 sont montés sur un support 213, par exemple une carte de circuit imprimé. Les émetteurs 211 peuvent par exemple être disposés sur une même face d'une carte de circuit imprimé, ou sur les deux faces opposées d'une carte de circuit imprimé comme cela est représenté en figure 2. Les émetteurs 211 peuvent être identiques ou similaires et commandables simultanément. A titre de variante, la source de lumière 103 comprend des émetteurs 211 présentant des propriétés distinctes, par exemple des bandes de longueurs d'ondes d'émission distinctes. De plus, les émetteurs 211 de la source de lumière 103 peuvent être commandables individuellement. A titre d'exemple, la source de lumière 103 comprend des émetteurs 211 adaptés à émettre dans une bande de longueurs d'ondes comprise dans la plage allant de 650 à 1100 nm. Plus généralement, toute autre gamme de longueurs d'ondes d'émission peut être prévue, par exemple la gamme allant de 400 à 650 nm pour des applications d'optogénétique.

La sonde 101 peut en outre comporter, à l'intérieur du tube 201, un capteur 215 adapté à surveiller l'herméticité du boîtier formé par le tube 201 et les bouchons 203 et 205. Dans l'exemple représenté, le capteur 215 est monté sur le même support 213 que les émetteurs 211 de la source lumineuse 103. Le capteur d'herméticité 215 comprend par exemple un capteur de la pression interne du tube 201, ou un capteur du taux d'humidité à l'intérieur du tube 201.

A titre de variante, le capteur 215 comprend une ou plusieurs diodes électroluminescentes organiques (OLED). Les diodes électroluminescentes organiques sont en effet connues pour voir leurs performances se dégrader en présence d'humidité ou d'oxygène. Dans cet exemple, on prévoit de mesurer des caractéristiques d'une ou plusieurs diodes électroluminescentes organiques pour détecter la présence éventuelle d'oxygène ou d'humidité dans des proportions anormales à l'intérieur du boîtier formé par le tube 201 et les bouchons 203 et 205, et en déduire la présence d'une fuite dans le boîtier. En particulier, en présence d'humidité ou d'oxygène, le rendement lumineux d'une diode électroluminescente organique décroit relativement rapidement. Ainsi, le capteur 215 peut comporter une diode électroluminescente organique et un capteur photosensible adapté à mesurer l'intensité lumineuse émise par cette diode. Le module d'alimentation et de commande 105 peut alors être adapté, à partir des mesures fournies par le capteur photosensible, à détecter une éventuelle fuite dans le boîtier formé par le tube 201 et les bouchons 203 et 205. Il est en outre connu que l'impédance d'une diode électroluminescente organique augmente relativement rapidement en présence d'humidité ou d'oxygène. Ainsi, le capteur 215 peut comporter une diode électroluminescente organique, le module 105 étant adapté à mesurer l'impédance de cette diode et à détecter, à partir des mesures d'impédance réalisées, une éventuelle fuite dans le boîtier formé par le tube 201 et les bouchons 203 et 205. On notera que dans le cas où le capteur 215 comprend une diode électroluminescente organique, cette diode peut être utilisée non seulement pour la surveillance d'étanchéité du boîtier formé par le tube 201 et les bouchons 203 et 205, mais aussi comme émetteur de lumière pour l'irradiation optique du cerveau du patient. Dans le cas où le capteur 215 comprend un capteur photosensible, ce dernier peut en outre être utilisé pour surveiller les signaux lumineux d'irradiation émis par les émetteurs 211.

Lorsque le module 105 détecte, à partir des mesures fournies par le capteur 215, une fuite dans le boîtier formé par le tube 201 et les bouchons 203 et 205, il déclenche par exemple une alarme pour signaler que la sonde 101 doit être remplacée.

La sonde 101 comprend en outre des éléments de connexion électrique 219 traversant le boîtier formé par le tube 201 et les bouchons 203 et 205 et reliant électriquement à l'extérieur du boîtier les composants situés dans le tube 201. Plus particulièrement, la sonde 101 comprend au moins deux éléments de connexion électrique 219 isolés reliés respectivement à une borne d'alimentation positive et à une borne d'alimentation négative de la source de lumière 103. D'autres éléments de connexion 219 peuvent en outre être prévus pour l'alimentation des composants situés à l'intérieur du tube 201 et/ou pour la communication avec ces composants. Dans l'exemple représenté, chaque élément de connexion électrique 219 comprend une tige conductrice traversant de part en part le bouchon 205 par l'intermédiaire d'un trou ou via percé dans ce bouchon. Chacun des vias est fermé hermétiquement par une brasure 221 en un matériau biocompatible, par exemple une brasure à l'or. La partie extérieure des éléments de connexion 219 peut être totalement ou partiellement noyée dans une goutte de résine 223 déposée sur la face extérieure du bouchon 205, de façon à renforcer la résistance mécanique de la zone d'interconnexion. Les éléments de connexion électrique 219 peuvent être en un matériau conducteur biocompatible, par exemple du platine iridié.

La sonde 101 comprend de plus, à l'extérieur du tube 201, un câble de liaison 225 reliant électriquement les éléments de connexion 219 à l'extrémité proximale 101b de la sonde. Le câble 225 comprend une pluralité de brins conducteurs isolés 227 connectés respectivement aux différents éléments de connexion 219. Les brins 227 peuvent être réalisés en un matériau conducteur biocompatible, par exemple en acier inoxydable.

Dans l'exemple de la figure 2, la sonde 101 comprend de plus une gaine de protection 229 en un matériau biocompatible transparent enrobant le tube 201 et le câble de liaison 227. La gaine 229 est par exemple en un matériau élastomère transparent, par exemple en silicone ou en polyuréthane. A titre d'exemple, la gaine 229 est en matériau présentant un module de Young compris entre 1 et 1000 MPa de façon à conférer une certaine flexibilité à la sonde 101.

Pour limiter les réflexions parasites de la lumière émise par la source 103, les parois internes du tube 201 peuvent être revêtues d'une couche antireflet. A titre de variante, un matériau de remplissage transparent d'indice adapté, par exemple un matériau d'indice inférieur à celui du matériau du tube 201, par exemple du silicone, peut être injecté à l'intérieur du tube 201. Le matériau de remplissage peut notamment être choisi pour avoir une conductivité thermique supérieure à celle de l'air. Ainsi, un avantage supplémentaire est que la présence du matériau de remplissage permet d'améliorer la dissipation de la chaleur produite par la source lumineuse, et d'améliorer en conséquence la durée de vie des composants.

Un avantage du dispositif décrit en relation avec les figures 1 et 2 réside dans le fait qu'il permet de mettre en oeuvre des stimulations optiques du cerveau plus variées qu'avec un dispositif à fibre optique. En particulier, le dispositif décrit permet d'irradier des surfaces plus importantes du cerveau qu'un dispositif à fibre optique. En outre, la source lumineuse 103 peut comporter des émetteurs 211 émettant simultanément dans des bandes de longueurs d'ondes distinctes.

De plus, la disposition de la source lumineuse à l'extrémité distale de la sonde 101, c'est-à-dire directement en vis-à-vis des régions à stimuler, permet de réduire la puissance de la source lumineuse et les pertes par couplage (présentes dans le cas d'un dispositif comprenant une source déportée et une fibre optique) et par conséquent la consommation électrique du dispositif par rapport à un dispositif à fibre optique.

Un autre avantage du dispositif décrit en relation avec les figures 1 et 2 est que la gaine d'enrobage peut être agencée de façon à conférer à la sonde 101 une forme générale et des propriétés mécaniques similaires à celles de dispositifs de stimulation cérébrale profonde électrique existants, ou à celles des dispositifs de stimulation cérébrale profonde optique à fibre optique existants. Ainsi, l'implantation de la sonde 101 peut être réalisée au moyen d'outils et de techniques chirurgicales déjà éprouvés. En outre, ce système reste compatible avec les éléments de contrôle de stimulation standard et la connectique associée.

La figure 3 représente une variante de réalisation du dispositif décrit en relation avec les figures 1 et 2. La figure 3 représente plus particulièrement la partie distale de la sonde 101 du dispositif.

La sonde 101 de la figure 3 comprend les mêmes éléments que la sonde 101 de la figure 2, agencés sensiblement de la même manière. La sonde 101 de la figure 3 diffère de la sonde 101 de la figure 2 en ce qu'elle comporte en outre, au niveau de sa partie distale, des électrodes 301 (quatre dans l'exemple représenté) adaptées à la mise en oeuvre d'une stimulation électrique profonde du cerveau. Les électrodes 301 sont disposées sur la périphérie extérieure de la gaine 229 et sont destinées à être placées directement en contact avec les tissus du cerveau. Les électrodes 301 ont par exemple la forme d'anneaux entourant la gaine 229. Chaque électrode 301 a par exemple une largeur (c'est-à-dire une distance dans l'axe longitudinal de la sonde 101) comprise entre 0,5 et 3 mm, par exemple de l'ordre de 1 mm. La distance (dans l'axe longitudinal de la sonde 101) entre deux électrodes 301 voisines est par exemple comprise entre 0,2 et 2 mm, par exemple de l'ordre de 0,5 mm. Les électrodes 301 sont par exemple disposées en amont de la sonde par rapport à la source lumineuse 103. A titre d'exemple, la distance (dans l'axe longitudinal de la sonde 101) entre la source lumineuse 103 et l'électrode 301 la plus proche de la source lumineuse 103 est comprise entre 0,5 mm et 1 cm, par exemple de l'ordre de 1 mm. Dans l'exemple de la figure 3, le câble de liaison 225 reliant la partie distale de la sonde 101 à son extrémité proximale 101b comprend en outre des brins conducteurs isolés 303 connectés respectivement aux différentes électrodes 301.

Dans l'exemple de la figure 3, le module de commande 105 (figure 1) du dispositif est non seulement adapté à commander la source lumineuse 103 pour mettre en oeuvre des stimulations optiques du cerveau, mais est en outre adapté à commander les électrodes 301 (par l'intermédiaire des brins conducteurs 303) pour mettre en oeuvre des stimulations électriques du cerveau, par exemple simultanément à la mise en oeuvre des stimulations optiques, ou en alternance avec les stimulations optiques.

Des modes de réalisation particuliers ont été décrits. Diverses variantes et modifications apparaîtront à l'homme de l'art. En particulier, les modes de réalisation décrits ne se limitent pas aux exemples de matériaux et de dimensions susmentionnés.

En outre, on a décrit ci-dessus un exemple de réalisation dans lequel le tube 101 est un tube initialement ouvert à ses deux extrémités, des bouchons 203 et 205 étant utilisés pour fermer hermétiquement les deux extrémités du tube. A titre de variante, le tube 101 peut être initialement ouvert à une seule de ses extrémités, auquel cas un seul bouchon peut être utilisé pour fermer le tube.

Par ailleurs, les modes de réalisation décrits ne sont pas limités à l'exemple susmentionné d'application à l'irradiation profonde du cerveau. A titre de variante, les dispositifs décrits peuvent être utilisés pour la stimulation profonde d'organes du corps humain ou animal autres que le cerveau.

## Revendications

1. Dispositif implantable pour la stimulation optique d'un organe du corps humain ou animal, comportant une sonde (101) comprenant :
un tube (201) en un premier matériau transparent, ce tube étant hermétiquement fermé par au moins un bouchon (203, 205) ;
au moins une source lumineuse (103) disposée à l'intérieur du tube (201) ;
une pluralité d'éléments de connexion électrique (219) traversant le bouchon (203 ; 205) et reliant électriquement ladite au moins une source lumineuse (103) à l'extérieur du tube (201) ;
un câble de liaison (225) disposé à l'extérieur du tube (201), connecté audits éléments de connexion (219) ;
**caractérisé en ce que** le dispositif comprend un capteur (215) adapté à détecter une éventuelle fuite dans le tube (201).

2. Dispositif selon la revendication 1, comportant en outre une gaine (229) en un deuxième matériau transparent enrobant le tube (201) et le câble (225).

3. Dispositif selon la revendication 2, dans lequel le deuxième matériau est un matériau élastomère.

4. Dispositif selon l'une quelconque des revendications 1 à 3, dans lequel le premier matériau est du saphir ou de la silice.

5. Dispositif selon l'une quelconque des revendications 1 à 4, dans lequel la sonde (101) a une forme longiligne, le tube (201) étant situé à une première extrémité (101a) de la sonde (101) et le câble de liaison (225) reliant la source lumineuse à une deuxième extrémité (101b) de la sonde (101) opposée à la première extrémité (101a).

6. Dispositif selon l'une quelconque des revendications 1 à 5, comportant en outre un module (105) d'alimentation et de contrôle relié électriquement à la source lumineuse (103) par l'intermédiaire du câble de liaison (225).

7. Dispositif selon l'une quelconque des revendications 1 à 6, dans lequel la source lumineuse (103) comprend une ou plusieurs diodes électroluminescentes (211).

8. Dispositif selon l'une quelconque des revendications 1 à 7, dans lequel ledit capteur (215) comprend une diode électroluminescente organique située à l'intérieur du tube (201).

9. Dispositif selon la revendication 8, dans lequel ledit capteur (215) comprend en outre un circuit de mesure d'une grandeur représentative de l'impédance de la diode électroluminescente organique et/ou du rendement lumineux de la diode électroluminescente organique.

10. Dispositif selon l'une quelconque des revendications 1 à 9, dans lequel un matériau de remplissage transparent est disposé à l'intérieur du tube (201).

11. Dispositif selon l'une quelconque des revendications 1 à 10, dans lequel la sonde (101) comprend en outre des électrodes (301) adaptées à la mise en oeuvre d'une stimulation électrique d'un organe du corps humain ou animal.

## Patentansprüche

1. Implantierbare Vorrichtung zur optischen Stimulation eines Organs des menschlichen oder tierischen Körpers, aufweisend eine Sonde (101), die Folgendes aufweist:
ein Rohr (201) aus einem ersten transparenten Material, wobei das Rohr durch wenigstens eine Verschlusskappe (203, 205) hermetisch verschlossen ist;
wenigstens eine Lichtquelle (103), die innerhalb des Rohres (201) angeordnet ist;
eine Vielzahl von elektrischen Verbindungselementen (219), die sich durch die Verschlusskappe (203; 205) erstrecken und die mindestens eine Lichtquelle (103) außerhalb des Rohres (201) elektrisch koppeln;
ein außerhalb des Rohres (201) angeordnetes Verbindungskabel (225), das mit den Verbindungselementen (219) verbunden ist; **dadurch gekennzeichnet, dass** die Vorrichtung einen Sensor (215) aufweist, der in der Lage ist, ein mögliches Leck in dem Rohr (201) zu detektieren.

2. Vorrichtung nach Anspruch 1, die ferner eine Hülle (229) aus einem zweiten transparenten Material aufweist, die das Rohr (201) und das Kabel (225) beschichtet.

3. Vorrichtung nach Anspruch 2, wobei das zweite Material ein Elastomermaterial ist.

4. Vorrichtung nach einem der Ansprüche 1 bis 3, wobei das erste Material Saphir oder Silika ist.

5. Vorrichtung nach einem der Ansprüche 1 bis 4, wobei die Sonde (101) eine längliche Form besitzt, wobei das Rohr (201) an einem ersten Ende (101a) der Sonde (101) angeordnet ist und wobei das Verbindungskabel (225) die Lichtquelle mit einem zweiten Ende (101b) der Sonde (101) entgegengesetzt zum ersten Ende (101a) verbindet.

6. Vorrichtung nach einem der Ansprüche 1 bis 5, die ferner eine Stromversorgungs- und Steuereinheit (105) aufweist, die über das Verbindungskabel (225) elektrisch mit der Lichtquelle (103) gekoppelt ist.

7. Vorrichtung nach einem der Ansprüche 1 bis 6, wobei die Lichtquelle (103) eine oder eine Vielzahl von lichtemittierenden Dioden (211) aufweist.

8. Vorrichtung nach einem der Ansprüche 1 bis 7, wobei der Sensor (215) eine organische lichtemittierende Diode aufweist, die innerhalb des Rohrs (201) angeordnet ist.

9. Vorrichtung nach Anspruch 8, wobei der Sensor (215) ferner eine Schaltung zum Messen einer Größe aufweist, die die Impedanz der organischen lichtemittierenden Diode und/oder die Lichteffizienz der organischen lichtemittierenden Diode repräsentiert.

10. Vorrichtung nach einem der Ansprüche 1 bis 9, wobei ein transparentes Füllmaterial innerhalb des Rohres (201) angeordnet ist.

11. Die Vorrichtung nach einem der Ansprüche 1 bis 10, wobei die Sonde (101) ferner Elektroden (301) aufweist, die in der Lage sind, eine elektrische Stimulation eines Organs des menschlichen oder tierischen Körpers zu implementieren.

## Claims

1. An implantable device for the optical stimulation of an organ of the human or animal body, comprising a probe (101) comprising:
a tube (201) made of a first transparent material, the tube being hermetically closed by at least one cap (203, 205);
at least one light source (103) arranged inside of the tube (201);
a plurality of electric connection elements (219) running through the cap (203; 205) and electrically coupling said at least one light source (103) outside of the tube (201);
a connecting cable (225) arranged outside of the tube (201), connected to said connection elements (219); **characterized in that** the device comprises a sensor (215) capable of detecting a possible leakage in the tube (201).

2. The device of claim 1, further comprising a sheath (229) made of a second transparent material coating the tube (201) and the cable (225).

3. The device of claim 2, wherein the second material is an elastomeric material.

4. The device of any of claims 1 to 3, wherein the first material is sapphire or silica.

5. The device of any of claims 1 to 4, wherein the probe (101) has an elongated shape, the tube (201) being located at a first end (101a) of the probe (101) and the connecting cable (225) coupling the light source to a second end (101b) of the probe (101) opposite to the first end (101a).

6. The device of any of claims 1 to 5, further comprising a power supply and control unit (105) electrically coupled to the light source (103) via the connecting cable (225).

7. The device of any of claims 1 to 6, wherein the light source (103) comprises one or a plurality of light-emitting diodes (211) .

8. The device of any of claims 1 to 7, wherein said sensor (215) comprises an organic light-emitting diode located inside of the tube (201).

9. The device of claim 8, wherein said sensor (215) further comprises a circuit for measuring a quantity representative of the impedance of the organic light-emitting diode and/or of the light efficiency of the organic light-emitting diode.

10. The device of any of claims 1 to 9, wherein a transparent filling material is arranged inside of the tube (201).

11. The device of any of claims 1 to 10, wherein the probe (101) further comprises electrodes (301) capable of implementing an electric stimulation of an organ of the human or animal body.
